# EUROPEAN PATENT SPECIFICATION

(11) **EP 2 639 237 B1**
(45) Date of publication and mention of the grant of the patent: **05.10.2016**
(21) Application number: 12194214.8
(22) Date of filing: 26.11.2012
(51) Int. Cl.: C07F 9/36, C07C 39/14, C07C 59/11

(54) **A process for the manufacture of optically active phosphinamide**
Ein Prozess für die Herstellung von optisch aktiven phosphinamide
Un procédé de fabrication de phosphinamide optiquement actif

(30) Priority: 12.03.2012 PL 39842212
(43) Date of publication of application: 18.09.2013
(73) Proprietor: Centrum Badan Molekularnych I Makromolekularnych Pan, 90-363 Lodz (PL)
(72) Inventor: Drabowicz, Jozef, 93-419 odz (PL); Lopusinski, Andrzej, 95-082 Dobron (PL); Krasowska, Dorota, 92-517 Lodz (PL)
(74) Representative: Brodowska, Iwona

(56) References cited:
- IRENE NOTAR FRANCESCO ET AL: "Stereoselective Addition of Grignard Reagents to New P-Chirogenic N-Phosphinoylbenzaldimines: Effect of the Phosphorus Substituents on the Stereoselectivity", EUROPEAN JOURNAL OF ORGANIC CHEMISTRY, vol. 2011, no. 20-21, 1 July 2011 (2011-07-01), pages 4037-4045, XP055055907, ISSN: 1434-193X, DOI: 10.1002/ejoc.201100380
- DRABOWICZ J ET AL: "New procedures for the resolution of chiral tert-butylphenylphosphine oxide and some of its reactions", TETRAHEDRON ASYMMETRY, PERGAMON PRESS LTD, OXFORD, GB, vol. 10, no. 14, 16 July 1999 (1999-07-16) , pages 2757-2763, XP004177237, ISSN: 0957-4166, DOI: 10.1016/S0957-4166(99)00264-5

## Description

The subject matter of the invention is a new process for the manufacture of optically active *t*-butyl-phenyl phosphinamide through the optical resolution of the racemate thereof using a reaction which involves formation of supramolecular complexes with enantiomerically pure mandelic acid or enantiomerically pure 2,2'-dihydroxy-1,1'-binaphthyl (BINOL).

A rapidly growing number of examples have been recently provided which suggest that the application properties of one of the enantiomers of a specific chemical structure are much more favourable than those of the racemic mixture or of the enantiomer with the opposite configuration [*a)* Chirality in Industry, Editors A. N. Collins, G N. Sheldrake, J. Crosby, Wiley & Sons, New York, 1997 *b)* Principle of Asymmetric Synthesis, R.E. Gowley, J. Aube, Pergamon, Oxford, 1996 *c)* Chiral Auxiliaries and Ligands in Asymmetric Synthesis, J. Seyden-Penne, VCH, 1996]. Therefore, the synthesis of optically active compounds is one of the most important issues currently faced by organic synthesis. In particular, the quest for new asymmetric synthesis procedures, including new chiral auxiliary agents needed for asymmetric synthesis, is the object of extensive studies by a number of groups, both academic and working for chemical industry, particularly pharmaceutical industry. This is due to the fact that asymmetric synthesis in most cases provides the most convenient and economically the most efficient solutions. [Comprehensive Asymmetric Catalysis: Editors: E. J. Jacobsen, A. Pfaltz, H. Yamamoto, Springer, Berlin, 1999*;* Comprehensive Asymmetric Synthesis: Editor I. Ojima, Wiley-VCH, New York, 1999). Chiral organic compounds with a stereogenic phosphorus atom are very commonly used as chiral ligands [Special Issue of Tetrahedron Asymmetry, 15, 2099 (2004) and subsequent papers] and organic catalysts [the most recent review - Org. Biomol. Chem., 8,3824 (2010); Acc. Chem. Res., 43, 1005 (2010)].

The formation of optically active *t*-butyl-phenyl phosphinamide of Formula **1** and unspecified enantiomeric excess based on the debenzoylation of *N-*benzylphosphinamide of Formula **5** in a hydrogenolysis reaction on palladium (10% Pd/C) in a mixture of formic acid and ethanol is reported in a paper published in 1996 (Tetrahedron Lett. Tetrahedron Lett. 1996, 37, 4733). The reactions involved conversion of an anion of optically active *t*-butyl-phenyl phosphine oxide **4** to respective *t*-butyl-phenyl phosphine bromide **6** conducted to yield *t*-butyl-phenyl-phosphino *N*-benzylamide **5.** Recently, the optically active phosphinamide was prepared by way of analogous chemical reactions involving optically active *t*-butyl-phenyl phosphine oxide of Formula **4** (resolved into its enantiomers through the formation of a complex with optically active mandelic acid, Tetrahedron: Asymmetry 1999, 10, 2757). The reductive elimination of the benzyl group from the amide [in conditions of pressure hydrogenolysis (20 bar/10% Pd/C) in ethanol] yielded optically active *t*-butyl-phenyl phosphinamide of Formula **1.** The optically active phosphinamide **1** prepared by way of chemical reactions was used for the synthesis of *t*-butyl-phenyl *N*-benzylidenephosphinamide **7** used as a substrate in the synthesis of a series of diastereomeric *N*-(α-alkyl)benzyl phosphinamides **8** (Chem. Commun., 2010, 2139; Eur. J. Org. Chem, 2011, 4037).

Optically active phosphinamide **1** prepared according to the present invention was in turn used for the synthesis of *t*-butyl-phenyl *N*-3-thienylphosphinamide **9** used as a substrate in the synthesis of a series of diastereomeric *N*-[(α-alkyl)(3-thienyl)]methyl phosphinamides of Formula **10.** The compound is used as a monomer in the polymerisation reaction which yields polythiophene systems functionalised at position 3 with a chiral phosphinylamide moiety. The polythiophenes obtained through the polymerisation contain a conjugated double bond system, being useful substrates for the manufacture of electronic materials with molecular dimensions. (J. Drabowicz, D. Krasowska; research in progress and a patent application being prepared for filing).

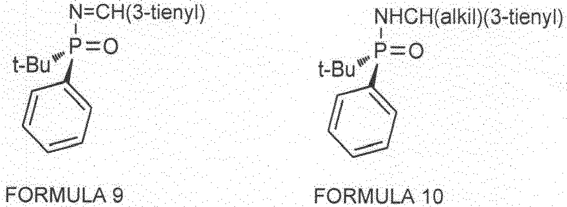

The process for the manufacture of optically active *t*-butyl-phenyl phosphinamide in the form of an enantiomerically pure levorotatory enantiomer of Formula **1** and absolute configuration (*R*) through the optical resolution of the racemate thereof according to the invention consists in that an enantiomerically pure dextrorotatory enantiomer of (*S*)-mandelic acid of Formula **2** or an enantiomerically pure dextrorotatory enantiomer of 2,2'-dihydroxy-1,1'-binaphthyl (BINOL) of Formula **3** and absolute configuration (*R*) is added to a solution of racemic amide, yielding a supramolecular complex of optically active levorotatory phosphinamide with configuration (*R*); subsequently, the resulting crystalline complex is filtered off and an optically active levorotatory phosphinamide with absolute configuration (*R*) is isolated in the process of the invention by treating separated crystals, having been dissolved in an organic solvent, preferably in the ethyl acetate medium, with a base, preferably solid sodium carbonate, to obtain chemically pure amide with 100% enantiomeric excess.

The process for the manufacture of optically active *t*-butyl-phenyl phosphinamide in the form of enantiomerically pure dextrorotatory *t*-butyl-phenyl phosphinamide of Formula **1** and absolute configuration (*S*) through the optical resolution of the racemate thereof according to the invention consists in that an enantiomerically pure levorotatory enantiomer of (*R*)-mandelic acid of Formula **2** or an enantiomerically pure levorotatory enantiomer of 2,2'-dihydroxy-1,1'-binaphthyl (BINOL) of Formula **3** and absolute configuration (*S*) is added to a solution of racemic amide, yielding a supramolecular complex of optically active dextrorotatory phosphinamide; subsequently, the resulting crystalline complex is filtered off and an optically active phosphinamide with absolute configuration (*S*) is isolated in the process of the invention by treating separated crystals, having been dissolved in an organic solvent, preferably in the ethyl acetate medium, with a base, preferably solid sodium carbonate, to obtain chemically pure amide with 100% enantiomeric excess. 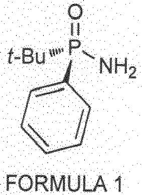 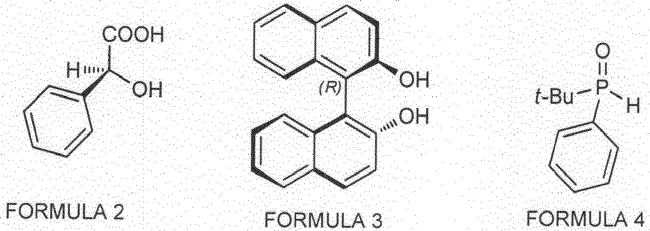 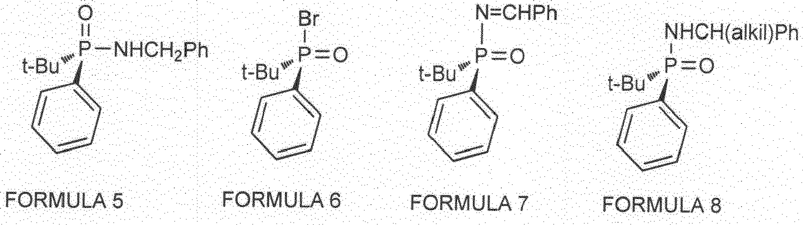 The processes for the manufacture of optically active *t*-butyl-phenyl phosphinamide of Formula **1** 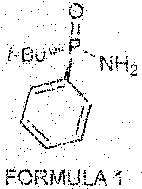 according to the invention consist in that a racemic mixture is treated with enantiomerically pure (+)-(*S*)-mandelic acid of Formula **2** 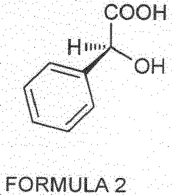 or enantiomerically pure (+)-(*R*)-2,2'-dihydroxy-1,1'-binaphthyl ((*R*)-BINOL) of Formula **3** 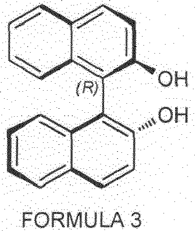 in organic solvents and the resulting crystals of the supramolecular complex whose breakdown enables the isolation of enantiomerically pure phosphinamide of Formula **1** are isolated. Optically pure phosphinamide **1** enriched with an enantiomer with an opposite absolute configuration at the stereogenic phosphorus atom is obtained from mother liquors following an appropriate isolation procedure.

The procedure for the resolution of racemic phosphinamide **1** through the formation of a complex with enantiomerically pure (-)-(*S*)-mandelic acid of Formula **2** is shown in **Scheme I.**

According to the Scheme, racemic phosphinamide **1** is treated with enantiomerically pure (-)-(*S*)-mandelic acid of Formula **2,** preferably in the diethyl ether medium at room temperature, for a period needed for the complex to precipitate completely and, subsequently, the resulting complex is filtered off and the levorotatory enantiomer of phosphinamide **1** is isolated by treating the separated crystals, having been dissolved in an organic solvent, preferably in the ethyl acetate medium, with a base, preferably solid sodium carbonate. After filtering off the base and evaporating the solvent, chemically pure amide with 100% enantiomeric excess is obtained, confirmed by chromatographic analysis using HPLC and a column with chiral packing. An oily residue is obtained when the diethyl ether is evaporated from the mother liquor from which the dextrorotatory enantiomer of phosphinamide **1** is isolated by treating the separated oil, having been dissolved in an organic solvent, preferably in the ethyl acetate medium, with a base, preferably solid sodium carbonate. After filtering off the base and evaporating the solvent, amide with approximately 40% enantiomeric excess is obtained, evaluated on the basis of its ¹H-NMR spectrum recorded in the presence of enantiomerically pure *t-*butylphenylthiophosphine acid as a chiral solvation reagent.

The procedure for the resolution of racemic phosphinamide **1** through the formation of a complex with enantiomerically pure (+)-(*R*)-2,2'-dihydroxy-1,1'-binaphthyl (BINOL) is shown in Scheme **II.**

According to the Scheme **II**, racemic phosphinamide **1** is treated with enantiomerically pure (+)-(*R*)-2,2'-dihydroxy-1,1'-binaphthyl (BINOL) of Formula **3,** preferably in the diethyl ether medium at room temperature for a period needed for the complex to precipitate completely and, subsequently, the resulting complex is filtered off and the levorotatory enantiomer of phosphinamide **1** is isolated by decomposing the resulting crystals by chromatography on a column with silica gel, preferably with 200-300 mesh granulation and using organic solvents as the eluent. Elution with acetone yields fractions from which the original (+)-(*R*)-2,2'-dihydroxy-1,1'-binaphthyl (BINOL) is isolated after solvent evaporation. Elution with methanol yields fractions from which after solvent evaporation phosphinamide with 100% enantiomeric excess is isolated, confirmed by chromatographic analysis using HPLC and a column with chiral packing.
The subject matter of the invention is illustrated by the following examples.

### Example I.

Enantiomerically pure (+)-(*S*)-mandelic acid **2** (1.52 g, 10 mmol) was added in three portions to a solution of racemic *t*-butyl-phenyl-phosphine amide **1** (1.97 g, 10 mmol) in diethyl ether (30 mL), stirred using a magnetic stirrer. After adding the last portion of mandelic acid, the reaction mixture was heated to a boil for 2 minutes and filtered hot. The filtrate was concentrated to a volume of 20 mL and left to stand at room temperature for 24 hours. Thereafter, the resulting crystalline complex was filtered off (0.68 g, 78 %). The compound is characterised by the following spectroscopy analysis data: [α]₅₈₉= + 76.5 (c= 1.39, CHCl₃), m.p. = 102-105 °C
¹H NMR (CDCl₃) =1.10 (d, J=16.97 Hz, 9H); 5.13 (s, 1H); 7.28- 7.49 (m 10H); 7.56 [t (broad), 1 H]; 7.82 [t (broad), 1H].
³¹P NMR (CDCl₃) = 46.73 (s).

The isolated complex was dissolved in dry ethyl acetate (35 mL) and solid, dry sodium carbonate (5 g) was added to the resulting solution. The resulting suspension was vigorously stirred for 72 hours using a magnetic stirrer. Subsequently, the precipitate was filtered off and washed with ethyl acetate (3x15 mL). When merged organic solutions were concentrated, initially at a pressure of 1999.8 Pa and subsequently at 133.32 Pa at room temperature, a chemically pure, levorotatory enantiomer of phosphinamide (-)- (*R*)- 1 was obtained (0.35 g, 35,7 %). The compound is characterised by the following spectroscopy analysis data:
[α]₅₈₉= -12.25 (c= 1.02, CHCl₃); m.p. = 59 - 60 °C [lit. for the racemate: m.p. = 85-86 °C (after chromatography and crystallisation from petroleum ether) - *J. Chem. Soc.,* 1975, 514]
¹H NMR (CDCl₃) =1.15 (d, J=15.28 Hz, 9H); 3.26 [s(broad), 2H]; 7.46-7.91 (m, 5H). The data are consistent with the literature data - J. Chem. Soc., 1975, 514. ³¹P NMR (CDCl₃) = 42.56 (s).
MS (Cl/isobutane): m/z: 198,1 [M+1]
Elemental analysis: for C₁₀H₁₆ NOP: calc.: C(60.86%), H(8.18%), N(7.14%); P (15.70); determined: C(60.92%), H(8.03%), N(7.25%); P(15.89)

### Example II.

A solution of (+)-(*R*)-2,2'-dihydroxy-1,1'-binaphthyl (BINOL) **3** (1.43 g, 5 mmol) in diethyl ether (30 mL) was added to a solution of racemic *t*-butyl-phenyl-phosphine amide **1** (0.98 g, 5 mmol). The reaction mixture was left to stand at room temperature for 72 hours. Thereafter, the precipitated complex was filtered off (0.821 g, 34 %). The compound is characterised by the following spectroscopy analysis data:
[α]₅₈₉= -23.7 (c= 1.16, CH₂Cl₂) ; m.p. = 178-179 °C
¹H NMR (CDCl₃) =1.14 (d, J=15.29 Hz, 9H); 2.73 [s (broad), 2H]; 5.35 [s (broad), 2H]; 7.14 (d, J= 8.4 Hz, 2H); 7.29-7.80 (m, 10H); 7.86-7.90 (m, 3H); 7.97 (d, J= 8.4 Hz, 2H)
³¹P NMR (CDCl₃) = 41.78 (s).

After two cycles of column chromatography of a part of the complex (0.230 g) on silica gel (20 g) enantiomerically pure (+)-(*R*)-2,2'-dihydroxy-1,1'-binaphthyl (BINOL) **3** was obtained after washing with acetone. Further washing with methanol yielded phosphinamide (-)-**1** (0.0828 g): [α]₅₈₉= -11.2 (c=2.48, CHCl₃), m.p. = 60-61 °C. Its 100% enantiomeric excess was confirmed by chromatographic analysis using HPLC and a column with chiral packing, and the structure was confirmed through proton and phosphorus magnetic resonance spectra and a mass spectrum, analogous to those specified in Example I.

## Claims

1. A process for the manufacture of an optically active form of *t*-butyl-phenyl phosphinamide in the form of an enantiomerically pure levorotatory enantiomer of Formula **1** and absolute configuration (R) 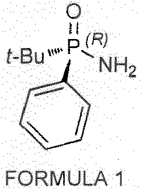 through the optical resolution of the racemate thereof, **characterised in that** an enantiomerically pure dextrorotatory enantiomer of (*S*)-mandelic acid of Formula **2** 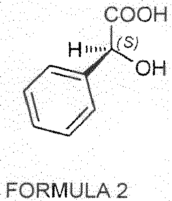 or an enantiomerically pure dextrorotatory enantiomer of 2,2'-dihydroxy-1,1'-binaphthyl (BINOL) of Formula 3 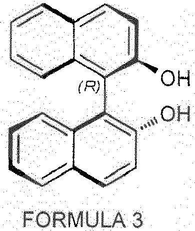 and absolute configuration (*R*) is added to a solution of racemic t-butyl-phenylphosphinamide, yielding a supramolecular complex of optically active levorotatory phosphinamide with configuration (*R*); subsequently, the resulting crystalline complex is filtered off and an optically active levorotatory phosphinamide with absolute configuration (*R*) is isolated therefrom ; whereby the resulting crystalline complex, is isolated by treating separated crystals, having been dissolved in an organic solvent, selected from a group consisting of methylene chloride, chloroform, diethyl ether, toluene, carboxylic acid esters, in particular in the ethyl acetate medium, with a base selected from a group of alkali metal salts, preferably solid sodium carbonate, to obtain chemically pure amide with 100% enantiomeric excess.

2. A process according to Claim 1, **characterised in that** the mandelic acid enantiomer of Formula **2** is used in an amount so that the molar ratio to *t*-butyl-phenyl phosphinamide **1** is at least 1:1.

3. A process according to Claim 1 or 2, **characterised in that** the 2,2'-dihydroxy-1,1'-binaphthyl (BINOL) enantiomer of Formula **3** is used in an amount so that the molar ratio to *t*-butyl-phenyl phosphinamide 1 is at least 1:1.

4. A process for the manufacture of optically active *t*-butyl-phenyl phosphinamide in the form of enantiomerically pure dextrorotatory *t*-butyl-phenyl phosphinamide of Formula **1** 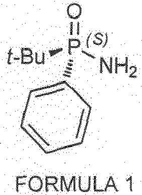 and absolute configuration (S) through the optical resolution of the racemate thereof, **characterised in that** an enantiomerically pure levorotatory enantiomer of (*R*)-mandelic acid of Formula **2** 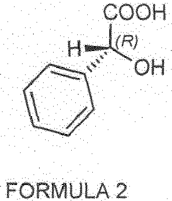 or an enantiomerically pure levorotatory enantiomer of 2,2'-dihydroxy-1,1'-binaphthyl (BINOL) of Formula **3** 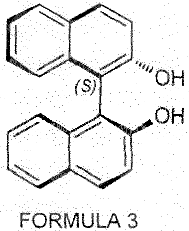 and absolute configuration (*S*) is added to a solution of racemic amide, yielding a supramolecular complex of optically active dextrorotatory phosphinamide; subsequently, the resulting crystalline complex is filtered off and an optically active phosphinamide with absolute configuration (*S*) is isolated therefrom; whereby the resulting crystalline complex is isolated by treating separated crystals, having been dissolved in an organic solvent, preferably in the ethyl acetate medium, with a base, preferably solid sodium carbonate, to obtain chemically pure amide with 100% enantiomeric excess.

5. A process according to Claim 4, **characterised in that** the mandelic acid enantiomer of Formula **2** is used in an amount so that the molar ratio to *t*-butyl-phenyl phosphinamide **1** is at least 1:1.

6. A process according to Claim 4 or 6, **characterised in that** the 2,2'-dihydroxy-1,1'-binaphthyl (BINOL) enantiomer of Formula **2** is used in an amount so that the molar ratio to *t*-butyl-phenyl phosphinamide **1** is at least 1:1.

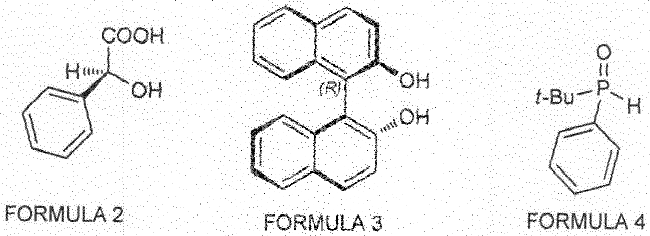

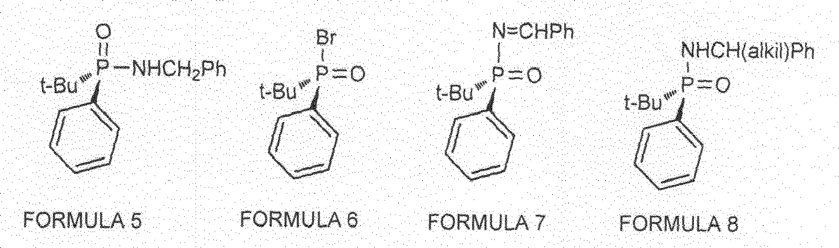

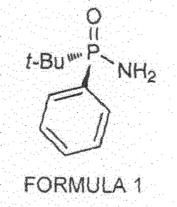

## Patentansprüche

1. Verfahren zur Herstellung von optisch aktiven Form von t-butyl-phenyl Phosphinamid in der Form von enantiomerenreinen linksdrehende Enantiomer der Formel 1 und die absolute Konfiguration (R) 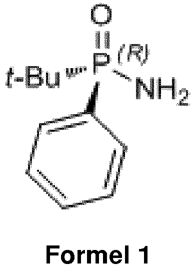 durch die optische Auflösung des Racemates, **dadurch gekennzeichnet, daß** einer enantiomerenreinen rechtsdrehenden Enantiomers von (S)-Mandelsäure der Formel 2 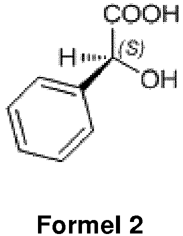 oder einer enantiomerenreinen rechtsdrehenden Enantiomer von 2,2'-dihydroxy-1,1'-binaphthyl (BINOL) der Formel 3 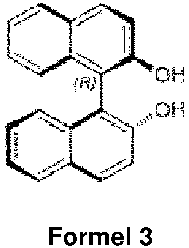 und die absolute Konfiguration (R) zu einer Lösung von racemisches t-Butyl-phenyl Phosphinamid zugesetzt wird, einen supramolekularen Komplex von optisch aktiven linksdrehenden Phosphinamid mit der Konfiguration (R) ergibt; anschließend das erhaltene kristalline Komplex abfiltriert und als optisch aktiver linksdrehender Phosphinamid mit der absoluten Konfiguration (R) aus ihr isoliert wird;. wodurch das resultierende kristalline Komplex durch Behandlung abgetrennten Kristalle isoliert ist, wurde in einem organischen Lösungsmittel gelöst, aus Methylenchlorid, Chloroform, Diethylether, Toluol, Carbonsäureester ausgewählt, insbesondere in Essigester Medium, mit einer Base aus einer Gruppe von Alkalimetallsalzen ausgewählt ist, vorzugsweise festem Natriumcarbonat, chemisch reine Amid mit der 100% Enantiomerenüberschuß zu erhalten.

2. Verfahren nach Anspruch 1, **dadurch gekennzeichnet, daß** die Mandelsäure Enantiomer der Formel 2 in einer Menge verwendet wird, so daß das Molverhältnis zu t-Butyl-phenyl Phosphinamid 1 mindestens 1:1 beträgt.

3. Verfahren nach Anspruch 1 oder 2, **dadurch gekennzeichnet, daß** das 2,2'-dihydroxy-1,1'-binaphthyl (BINOL) Enantiomer der Formel 3 in einer Menge verwendet wird, so daß das Molverhältnis zu t-Butyl-phenyl Phosphinamid 1 mindestens 1:1 beträgt.

4. Verfahren zur Herstellung von optisch aktiven t-Butyl-phenyl Phosphinamid in der Form von enantiomerenreinen rechtsdreh t-Butyl-phenyl Phosphinamid der Formel 1 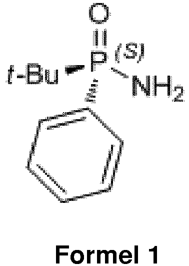 und die absolute Konfiguration (S) durch die optische Auflösung des Racemates, **dadurch gekennzeichnet, daß** ein enantiomerenreines linksdrehenden Enantiomer von (R)-Mandelsäure der Formel 2 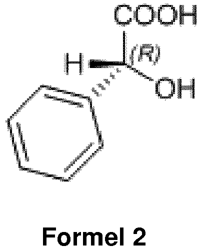 oder ein enantiomerenreines linksdrehenden Enantiomer von 2,2'-dihydroxy-1,1'-binaphthyl (BINOL) der Formel 3 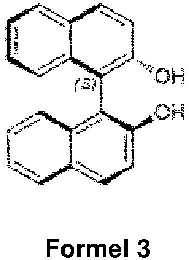 und die absolute Konfiguration (S) wird zu einer Lösung racemisches Amid zugesetzt, einen supramolekularen Komplex von optisch aktiven rechtsdrehender Phosphinamid ergibt; anschließend wird das erhaltene kristalline Komplex abfiltriert und ein optisch aktives Phosphinamid mit der absoluten Konfiguration (S) daraus isoliert; wodurch das resultierende kristalline Komplex durch Behandlung abgetrennten Kristalle isoliert ist, wurde in einem organischen Lösungsmittel gelöst, insbesondere in Essigester Medium, mit einer Base, vorzugsweise festem Natriumcarbonat, chemisch reine Amid mit der 100% Enantiomerenüberschuß zu erhalten.

5. Verfahren nach Anspruch 4, **dadurch gekennzeichnet, daß** die Mandelsäure Enantiomer der Formel 2 in einer Menge verwendet wird, so daß das Molverhältnis zu t-butyl-phenyl Phosphinamid 1 mindestens 1:1.

6. Verfahren nach Anspruch 4 oder 6, **dadurch gekennzeichnet, daß** das 2,2'-dihydroxy-1,1'-binaphthyl (BINOL) Enantiomer der Formel 2 in einer Menge verwendet wird, so daß das Molverhältnis zu t-butyl- phenyl Phosphinamid 1 mindestens 1:1.

## Revendications

1. La manière de recevoir une forme optiquement active de fosfinamide t-butyle-phényle sous la forme de l'énantiomère pur sénestre énantiomèriquement de la formule 1 et de la configuration absolue (R) 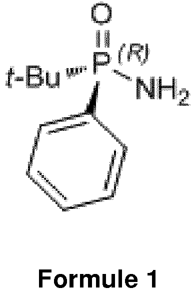 par la séparation optique de son racémate, **caractérisé en ce que** l'énantiomère pur sénestre énantiomèriquement de (*S*)-l'acide mandélique avec la formule 2 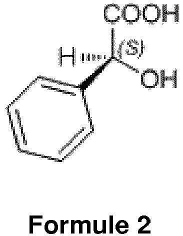 ou énantiomère pur sénestre énantiomèriquement 2,2'-dihydroxy-1,1'-binaphtyle (BINOL) avec la formule 3 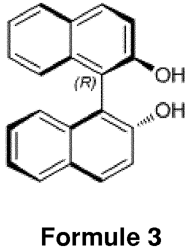 et la configuration absolue (R) est ajouté à la solution racémique du fosfinamide t-butyle-phényle, obtenant un complexe supramoléculaire du fosfinamide actif sénestre optiquement avec la configuration absolue (R); puis un complexe cristallin est vidangé et isolé en soumettant des cristaux distincts, dissous dans un solvant organique choisi dans le groupe composé de chlorure de méthylène, de chloroforme, d'éther, de toluène, d'esters d'acides carboxyliques, en particulier dans un environnement d'acétate d'éthyle, du principe de fonctionnement d'un groupe sélectionné de sels de métaux alcalins, carbonate de sodium solide et amide chimiquement pur à 100 % de l'excédent énantiomérique.

2. La manière selon la revendication 1, **caractérisé en ce qu'**un énantiomère de l'acide mandélique avec la formule 2 est utilisé en quantité suffisante aux molaires à fosfinamide *t*-butyle-phényle 1 soit égal au moins 1:1.

3. La manière selon la revendication 1 ou 2, **caractérisé en ce que** cet énantiomère 2,2'-dihydroxy-1,1'-binaphtyle (BINOL) avec la formule 3 est utilisé en quantité suffisante aux molaires à fosfinamide *t*-butyle-phényle 1 soit égal au moins 1:1.

4. Une méthode de production du fosfinamide optiquement actif t-butyle-phényle sous la forme pur dextrogyre énantiomèriquement du fosfinamide *t*-butyle-phényle avec la formule 1 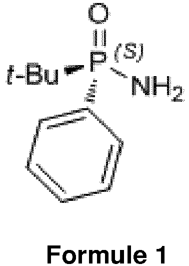 et la configuration absolue (S), par la séparation optique de son racémate, **caractérisé en ce que** l'énantiomère pur sénestre énantiomèriquement de (*R*)-l'acide mandélique avec la formule 2 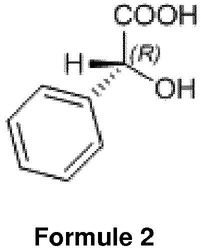 ou l'énantiomère pur sénestre énantiomèriquement 2,2'-dihydroxy-1,1'-binaphtyle (BINOL) avec la formule 3 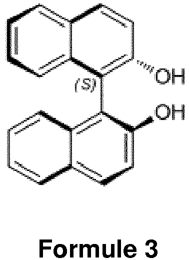 et la configuration absolue (S) est ajouté à une solution racémique de l'amide, obtenant un complexe supramoléculaire du fosfinamide actif dextrogyre optiquement; puis on vidange et isole un complexe cristalline qui en résulte du fosfinamide optiquement actif de configuration absolue (S); ainsi, un cristallin créé est isolé en soumettant des cristaux distincts, dissous dans un solvant organique, de préférence dans un environnement d'acétate d'éthyle, à l'action de la base, de préférence du carbonate de sodium et on obtient un amide chimiquement pur à 100 % de l'excédent énantiomérique.

5. La manière selon la revendication 4, **caractérisé en ce qu'**un énantiomère de l'acide mandélique avec la formule 2 est utilisé en quantité suffisante au rapport molaire à fosfinamide *t*-butyle-phényle 1 soit égal au moins 1:1.

6. La manière selon la revendication 4 ou 5, **caractérisé en ce que** cet énantiomère 2,2'-dihydroxy-1,1'-binaphtyle (BINOL) avec la formule 2 est utilisé en quantité suffisante pour que le rapport molaire à fosfinamide *t-*butyle-phényle éther 1 soit égal au moins 1:1.
